(19) **Europäisches Patentamt / European Patent Office / Office européen des brevets**

(11) **EP 4 365 304 B1**

(12) **EUROPEAN PATENT SPECIFICATION**

(45) Date of publication and mention
of the grant of the patent:
**04.06.2025 Bulletin 2025/23**

(21) Application number: **22383065.4**

(22) Date of filing: **07.11.2022**

(51) International Patent Classification (IPC):
**C12Q 1/6853** (2018.01)   **C12Q 1/6876** (2018.01)

(52) Cooperative Patent Classification (CPC):
(C-Sets available)
**C12Q 1/6853; C12Q 1/6876;** C12Q 2600/178
(Cont.)

(54) **A STEM-LOOP PRIMER AND A METHOD FOR SHORT LENGTH RNA DETECTION**

STAMMSCHLEIFEN-PRIMER UND VERFAHREN FÜR DEN NACHWEIS VON RNA KURZER LÄNGE

AMORCE TIGE-BOUCLE ET PROCÉDÉ DE DÉTECTION D'ARN DE COURTE LONGUEUR

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**08.05.2024 Bulletin 2024/19**

(73) Proprietor: **Universidad Autónoma de Madrid**
**28049 Madrid (ES)**

(72) Inventors:
• **Sánchez Pacheco, Aurora**
**28049 Madrid (ES)**
• **López López, Ana**
**28049 Madrid (ES)**
• **Camblor Murube, Marina**
**28049 Madrid (ES)**

(74) Representative: **Pons**
**Glorieta Rubén Darío 4**
**28010 Madrid (ES)**

(56) References cited:
**CN-A- 113 999 896**

• **VARKONYI-GASIC ERIKA ET AL: "Protocol: a highly sensitive RT-PCR method for detection and quantification of microRNAs", PLANT METHODS, BIOMED CENTRAL, LONDON, GB, vol. 3, no. 1, 12 October 2007 (2007-10-12), pages 12, XP021039366, ISSN: 1746-4811, DOI: doi.org/ 10.1186/1746-4811-3-12**
• **JUNG ULRIKE ET AL: "A universal TaqMan-based RT-PCR protocol for cost-efficient detection of small noncoding RNA", RNA, vol. 19, no. 12, 22 October 2013 (2013-10-22), US, pages 1864 - 1873, XP093039807, ISSN: 1355-8382, DOI: 10.1261/rna.040501.113**

Remarks:
The complete document including Reference Table(s) and the Sequence Listing(s) can be downloaded from the EPO website

**(Cont. next page)**

(52) Cooperative Patent Classification (CPC): (Cont.)

C-Sets
**C12Q 1/6853, C12Q 2525/207, C12Q 2525/301,
C12Q 2561/113**

**Description**

The invention relates to a novel stem-loop primer which presents a short length RNA target-specific region but also a universal region and a method for short length RNA detection, particularly miRNA detection, based on the combination of the novel stem-loop and a miRNA-specific forward primer with a universal reverse primer and universal probe in a reverse-transcription quantitative PCR (RT-qPCR).

**BACKGROUND ART**

MicroRNAs (miRNAs) are naturally occurring, highly conserved families of transcripts (18 to 25 nucleotides in length) that are processed from larger hairpin precursors. miRNAs are found in the genomes of animals and plants. To date, there are about 1000 unique transcripts, including 326 human miRNAs in the Sanger Center miRNA registry. miRNAs regulate gene expression by catalyzing the cleavage of messenger RNA (mRNA) or repressing mRNA translation. They are believed to be critical in cell development, differentiation and communication. Specific roles include the regulation of cell proliferation and metabolism, developmental timing, cell death, haematopoiesis, neuron development, human tumorigenesis and DNA methylation and chromatin modification. Main efforts have been made to identify the exact functions of each miRNA, focusing on their use in both research and clinical field.

Considering how the dysregulation of some miRNAs contribute to serious human diseases, such as some types of cancer, several detection methods including Northern blotting, cloning, and RT-qPCR have been developed to detect miRNA with high sensitivity and specificity, with real time RT-qPCR being considered as the gold standard for RNA quantification. Therefore, the RT-qPCR method for diagnostic purposes is proposed as the one with the biggest potential. However, the short sequence lengths, large variability in per-cell copy number, and high sequences similarity of expressed miRNAs compromise sensitivity and /or specificity of RT-qPCR methods.

Very short RNAs, including the microRNA, are not long enough to provide sufficient complementarity for conventional linear RT-PCR forward, reverse primers and fluorescence probe. In 2005, a TaqMan RT-PCR protocol was reported which uses stem-loop RT primers to specifically trap the 3' end of small RNAs (reducing the amount of complementarity required. The authors attribute the observed higher specificity and sensitivity of the stem-loop RT primers as compared with linear primers with base stacking and spatial constraint of the stem-loop structure (Chen C., et al, 2005, Nucleic Acids Research, Vol. 33, No. 20).

Kramer M.F., et al, 2011, Curr Protoc Mol Biol describes a specific and sensitive quantitative reverse-transcription PCR (RT-qPCR) method for measuring individual microARNs (miARNs) in tissue or cultured cells, including a RT stem-loop primer, the forward and reverse PCR primers, and the hydrolysis probe, contain design features that, together, optimize miARN specificity and assay sensitivity.

Ulrike Jung et al, 2013, RNA journal, Vol. 19, No. 12, discloses a universal TaqMan-based probe protocol which can be used to detect any target sequence and demonstrate its applicability for the detection of endogenous as well as artificial eukaryotic and bacterial small ARNs.

Varkonyi-Gasic et al., 2007, Plant Methods 3, 12, discloses a protocol for the sensitive detection of miRNAs from plant tissues. It involves using a stem-loop primer, a universal TaqMan probe, and universal reverse primers to detect short-length RNA.

CN113999896A discloses a universal stem-loop primer designed to detect short-length RNA. It demonstrates that a pool of stem-loop primers with a random hybridization region at the 3'-end can effectively detect miRNA in a sample.

However, an unspecific overlap between the hairpin region of the stem-loop primers and the specific region of some of the targeted miRNAs has been detected. Therefore, it must be taken into consideration when designing the amplification strategy.

The major current miRNA detection systems base their specificity on a combination of primers and fluorescent probes specific for each targeted miRNA. This entails the need of using a specific detection kit for each analysed miRNA. However, experiments performed in both research and clinical diagnosis fields require the analysis of complete panels of miRNAs, resulting into highly expensive experiments and leading researchers to limit their spectrum of analysis.

Therefore, it becomes necessary to develop stem-loop primers for a specific amplification without any unspecific overlapping with the miRNA and to design miRNA detection systems with excellent performance amplifying all the miRNAs tested.

**DESCRIPTION OF THE INVENTION**

The inventors have designed a novel primer stem-loop which contains the replacement of a guanine (G) for a cytosine (C) and 6 nucleotides short length RNA-primer specific region, increasing the specificity avoiding non-specific overlaps during the amplification process. The detection system shows an excellent performance amplifying short length RNAs, particularly miRNAs. After performing several initial experiments using the stem-loop primer with nucleotide sequence SEQ ID NO: 48 discloses in *Garcia López R., et al, 2018,* the inventors detected an unspecific overlap between the hairpin region and the specific region of some of the targeted miRNAs. In order to solve this problem, a modification was introduced in one of the bases located at the primer's hairpin (in 5'-3' sense, replacing the G at position 24 by a C of the nucleotide sequence SEQ ID NO: 48).

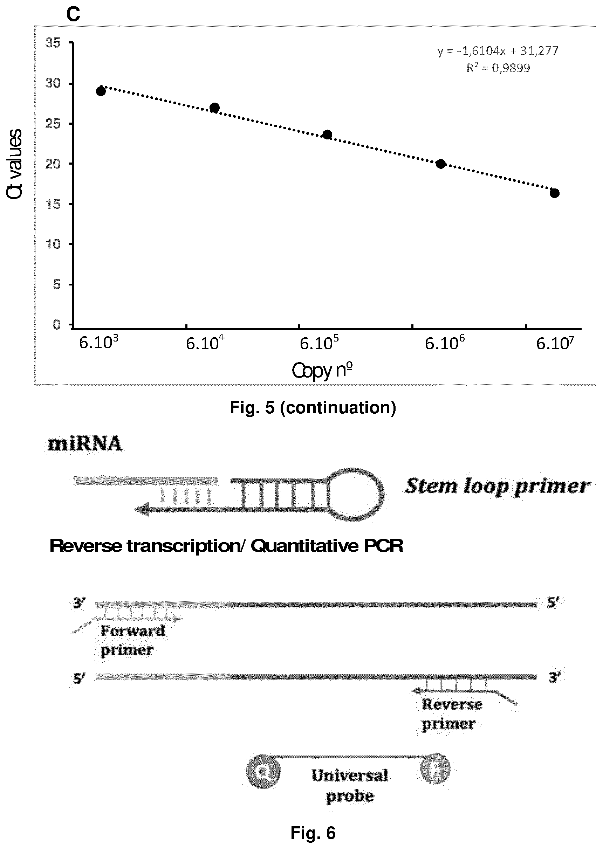

The method is not limited to miRNA detection, but it could be potentially applicable to amplify any kind of short length RNAs. So, the inventors have also proposed a short length RNAs detection method, preferably a miRNA detection method, based on the combination of the stem-loop and a specific forward primer with a universal reverse primer and a universal fluorescent probe in a reverse-transcription quantitative PCR (RT-qPCR) system for the specific detection of nucleic acid sequences. The stem-loop primer structure presents a 100% target-specific region but also a universal region, which fully hybridizes with both the universal probe and the universal reverse primer (Figure 6).

The aim of the proposed development is to design a short length RNAs detection system, preferably a miRNA detection system, in which the specificity relies exclusively on the forward and the stem-loop primer, allowing the use of a universal primer reverse and a universal probe for all the targeted short length RNAs, preferably miRNAs, without compromising the specificity of the system. By including a valid universal probe, which is the priciest element of regular amplification systems, the experiment expenses would be considerably reduced.

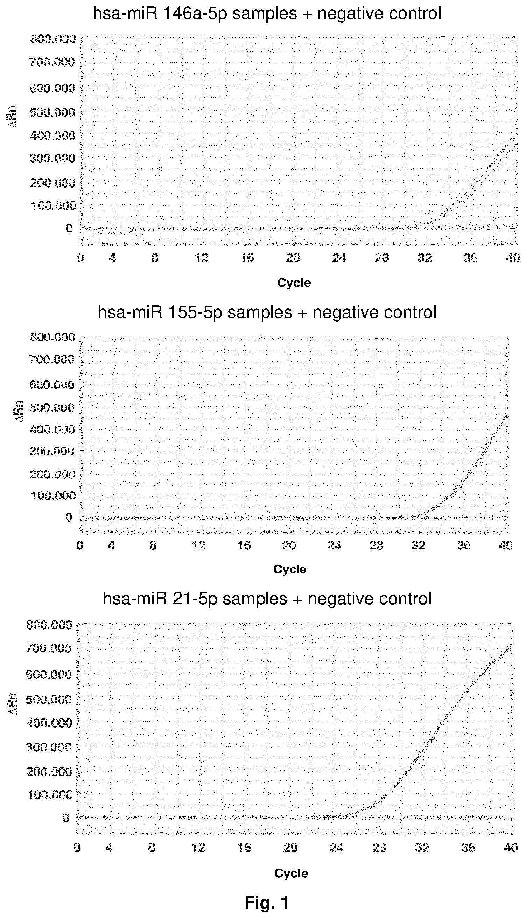

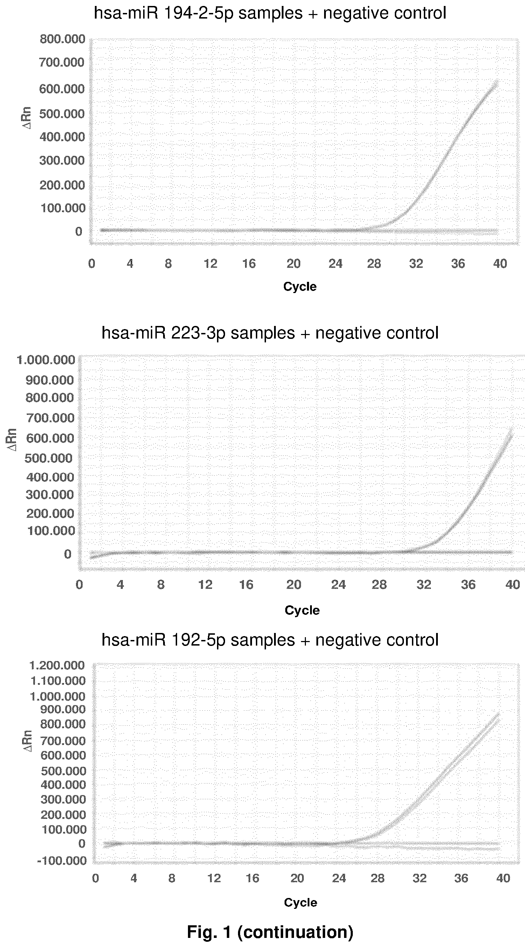

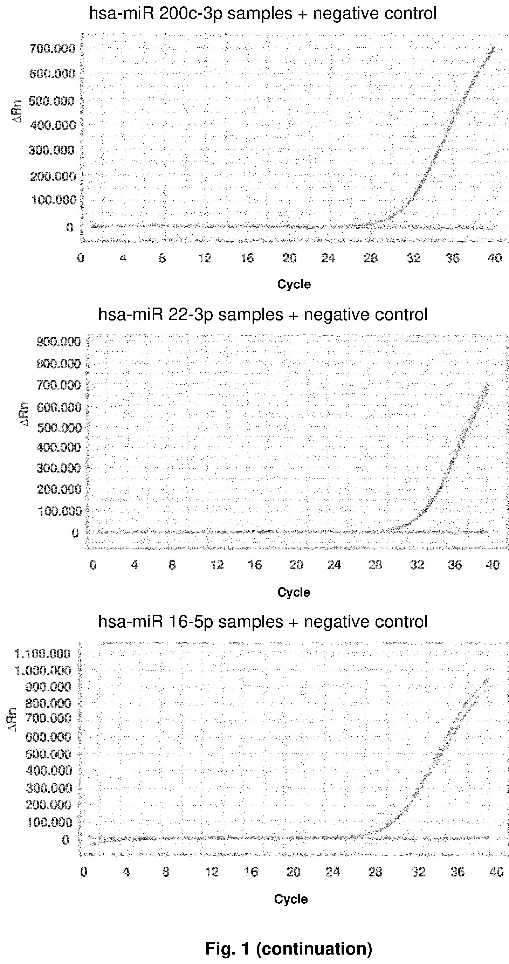

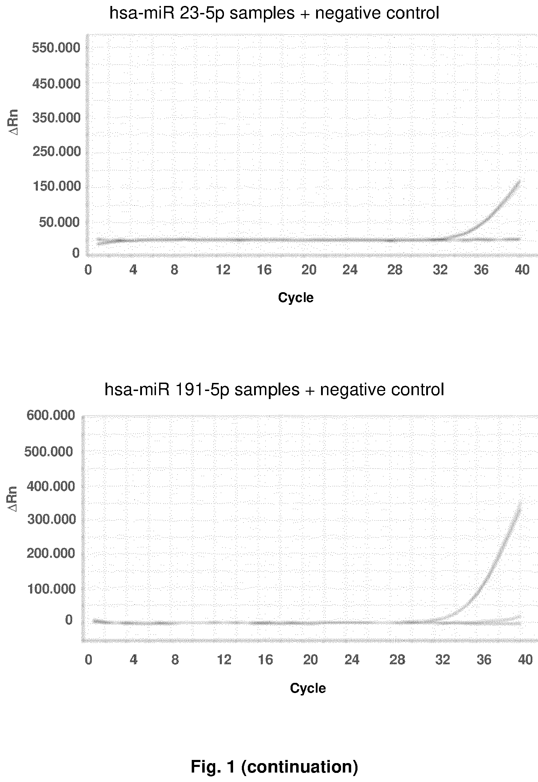

So far, the proposed method has been validated on a total of 40 human stool samples from patients with different types of cancer undergoing immunotherapy. The amplification replicates for both systems, validation in human stools samples and in Huh-7 cells, are almost identical, showing a high level of reproducibility using real biological samples as a substrate. Moreover, the method was tested in a small set of serum samples from patients with cancer, showing excellent results in terms of RT-qPCR performance as well (Table 10 and Figures 1, 2 and 3).

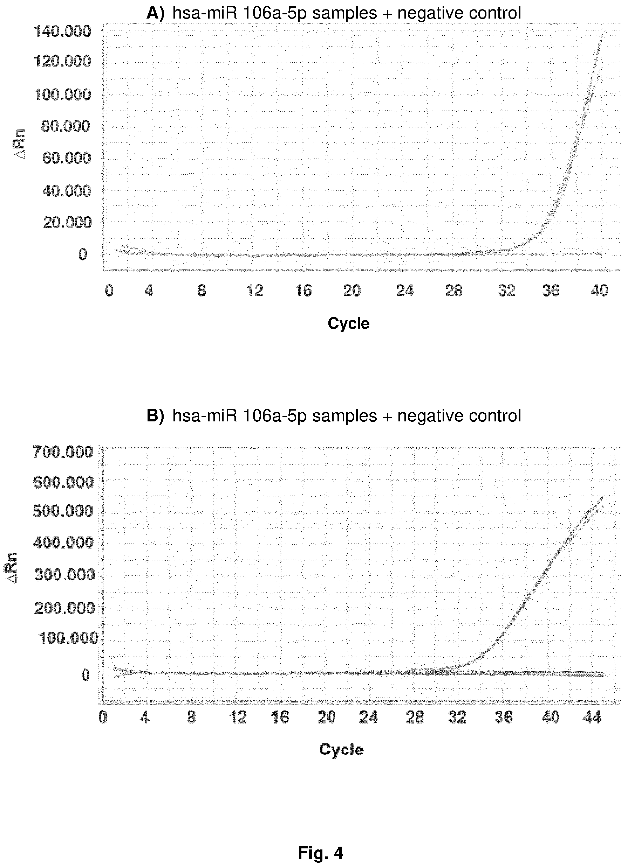

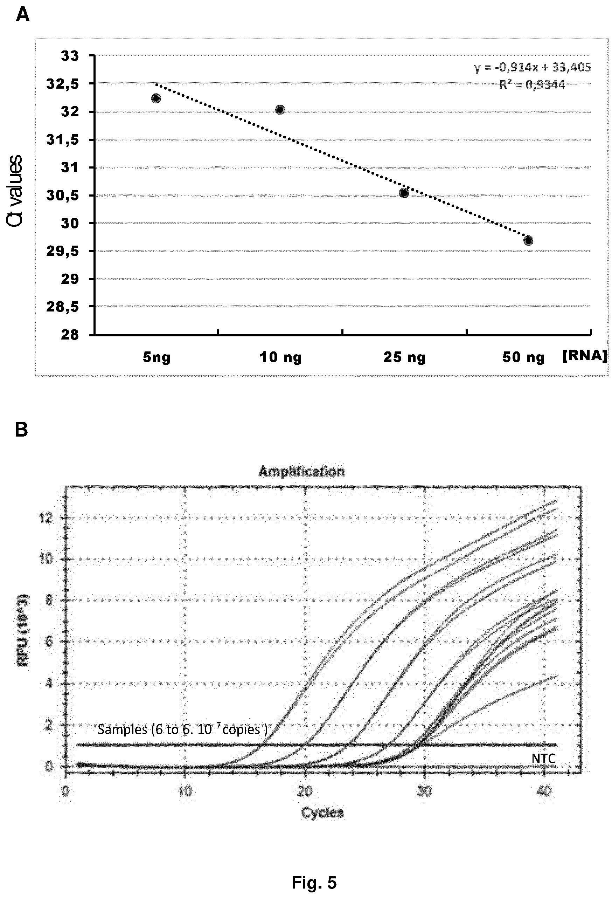

Finally, the detection kit developed by the inventors allows to generate qPCR amplification profiles similar to the ones obtained from the Applied Biosystem kit, even though the commercial kit uses one specific probe per targeted short length RNA, preferably per targeted miRNA. Once again, these results support a high specificity as one of the main strengths of this novel short length RNAs detection system (Figure 4). One additional advantage of this kit is the capacity to amplify up to 6 copies per reaction of substrate which allow to work with very small amounts of RNA (only 5 ng) while commercial kits use a minimal amount of 10 ng. This is important in the case of miRNAs amplification from RNA extracted from tissues of biopsied samples (Figure 5).

Thus, a first aspect of the present invention related to a stem-loop primer comprising the nucleotide sequence SEQ ID NO: 1, hereinafter the "stem-loop primer of the invention".

The term "stem-loop primer" as used herein refers to a primer which comprises a 3' target specific portion, a stem and a loop and that is designed to acquire a hairpin shape while leaving nucleotides exposed at its 3' end (Figure 6). The term "3' target-specific portion" refers to the single stranded portion of a stem-loop primer that is complementary to a target polynucleotide, preferably to a target short length RNA, more preferably to a microRNA or miRNA. The 3' target-specific portion can be between 5 and 9 nucleotides long, preferably the 3' target-specific portion is 6 nucleotides long. The term "stem" refers to the double stranded region of the stem-loop primer that is between the 3' target-specific portion and the loop. The term "loop" refers to a region of the stem-loop primer that is located between the two complementary strands of the stem and comprises single stranded nucleotides. Further, the stem-loop is a primer specifically designed to amplify a particular short length RNA, preferably a miRNA, and complementary to the said RNA, thus allowing its amplification by RT-qPCR (Reverse Transcription Quantitative Polymerase Chain Reaction).

The stem-loop of the invention comprises the nucleotide sequence SEQ ID NO: 1 which comprises a modification introduced in one of the bases located at the primer's hairpin of the stem-loop primer with nucleotide sequence SEQ ID NO: 48 discloses in *Garcia López R., et al, 2018,* particularly in 5'-3' sense, replacing the guanine (G) at position 24 by a cytosine (C) (G24C), wherein is marked in bold on the sequence SEQ ID NO: 1 below, and wherein the "NNNNNNN" is the short length RNA-specific region or 3' target-specific portion with a 6 nucleotides length and "N" comprises any nucleotide selected from the list consisting of: adenine or A, guanine or G, cytosine or C and thymine or T. The stem-loop primer of the invention also comprises a stem region contains 14 complementary nucleotides which are underlined:

SEQ ID NO: 1
5'-<u>GTTGGCTCTGGTGC</u>AGGGTCCGA**C**GTATT<u>CGCACCAGAGCCAAC</u>NNNNNNN-3'

SEQ ID NO: 48 5'-GTTGGCTCTGGTGCAGGGTCCGAGGTATTCGCACCAGAGCCAACNNNNNNNN-3'

In a preferred embodiment of the present invention, the stem-loop of the invention consisting of the nucleotide sequence SEQ ID NO: 1.

In a preferred embodiment of the present invention, the stem-loop of the invention consisting of the nucleotide sequence SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33 and any combination thereof.

As a person skilled in the art knows, the stem-loop primer of the invention can be included in a composition. So, other aspect of the invention relates to a composition comprising at least one stem-loop primer of the invention, hereinafter the "the composition of the invention".

As a person skilled in the art knows, the stem-loop primer in the composition of the invention can be lyophilized or diluted in nuclease-free water, for example in TE1x, preferably at a concentration of 20 micromolar.

As a person skilled in the art knows, for short length RNA detection, the stem-loop or the composition of the invention can be included in a kit. Thus, other aspect of the invention relates to a kit for short length RNA detection comprising at least one stem-loop primer or the composition of the invention, hereinafter "the kit of the invention".

As used herein, the term "kit" refers to a product comprising reagents necessary to carry out the short length RNA detection or amplification and which is adapted to permit the transport and storage of said reagents. Suitable materials for packaging the kit components may be made of plastic (such as polyethylene, polypropylene, polycarbonate and the like), glass, and may comprise bottles, flasks, sachets, etc. In addition, the kit of the invention may contain instructions for the simultaneous, sequential or separate use of the different components contained in the kit.

The term "reagents needed to detect or to amplify the short length RNA" means a compound or group of compounds that allow the short length RNA sequence to be determined. Examples of reagents needed to detect or quantify short length RNA include, but are not limited to, buffer, deoxyribonucleotide triphosphate (dNTP) mixtures [such as deoxyadenosine triphosphate (dATP), deoxyguanosine triphosphate (dGTP), deoxycytidine triphosphate (dCTP), deoxythymidine triphosphate (dTTP), and deoxyuridine triphosphate (dUTP)], reverse transcriptase, polymerase, enzyme cofactors, RNAase inhibitors, primers, conventional or real-time thermal cycler, and fluorophore probes (such as SYBR Green).

In a preferred embodiment of the present invention, the kit further comprises a reverse transcriptase.

The term "reverse transcriptase" refers to an enzyme that it synthesizes a DNA complementary to a template RNA, i.e., a DNA polymerase that can copy first-strand cDNA from an RNA template. Such enzymes are commonly referred to as RNA-directed DNA polymerases and have IUBMB activity EC 2.7.7.49.

In a preferred embodiment of the present invention, the kit is used for short length RNA detection, preferably for miRNA detection.

As knows the skilled person in the art, the kit for short length RNA detection or amplification can also include at least one forward primer, at least one reverse primer and/or at least one probe.

The term "forward primer" used herein refers to a primer that hybridizes (or anneals) with the template strand or antisense strand of the double DNA. The term "reverse primer" used herein refers to a primer that hybridizes (or anneals) to the complementary strand or sense strand of the double DNA. The forward primer hybridizes with the target sequence 5' with respect to the reverse primer.

It is routine practice for the skilled person in the art to use a primer set. As used herein, the expression "primer set" refers to two or more primers which together are capable of priming the amplification of a target sequence or target nucleic acid of interest (e.g., a target miRNA). In certain embodiments, the term "primer set" refers to a pair of primers including a 5' (upstream) primer (or forward primer) that hybridizes with the 5'-end of the target sequence or target nucleic acid to be amplified and a 3' (downstream) primer (or reverse primer) that hybridizes with the complement of the target sequence or target nucleic acid to be amplified. Such primer sets or primer pairs are particularly useful in PCR amplification reactions.

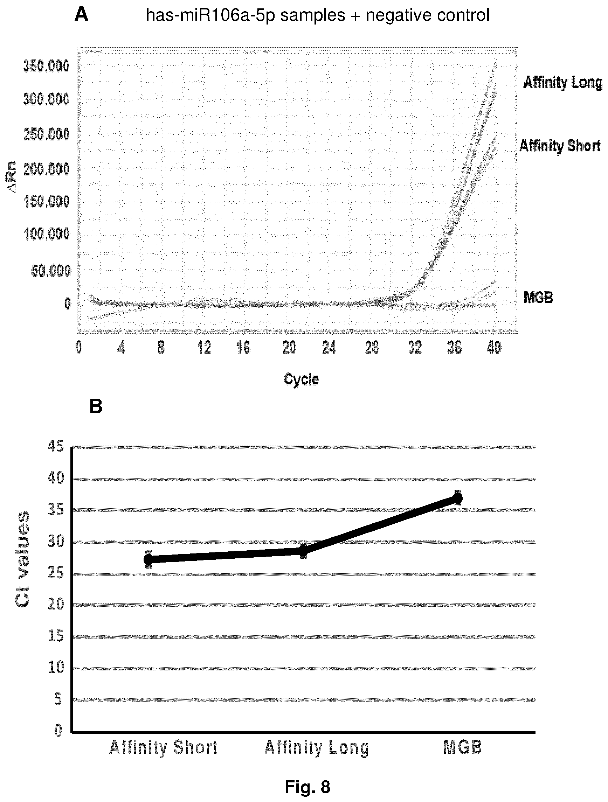

As used herein, the term "probe" refers to a molecule used in an amplification or detection reaction, typically for quantitative or real-time PCR analysis, as well as endpoint analysis. Examples of probes include, but are not limited to, MGB (Minor Groove Binder) and Affinity probes (Fig. 8).

In this invention the target short length RNA, preferably the target miRNA, is reverse transcribed into cDNA commonly using a stem-loop primer specific to the selected short length RNA, preferably selected miRNA. Afterwards, the cDNA obtained after reverse transcription of the short length RNA is usually amplified using a Taqman probe although any fluorescence probe might be used.

The inventors tested the hydrolysis Affinity Plus probe, similar to MGB probes. This type of probe includes a fluorescence dye at the 5'-end (Fluorescein amidites or FAM) and a quencher for the ligand binding type Iowa Black at the 3'-end. FAM is a fluorescent dye attachment for oligonucleotides and is compatible with most fluorescence detection equipment. It becomes protonated and has decreased fluorescence below pH 7; it is typically used in the pH range 7.5 to 8.5. Moreover, FAM can be attached to 5' or 3' end of oligos. Its absorbance range is from 495 nm to 520 nm (emission maximum).

The Affinity Plus technology is based on the introduction of locked nucleic acids (or LNAs). When incorporated into a probe, LNAs impart heightened structural stability, leading to increased hybridization melt temperature ($T_m$). An additional advantage of this probe is that it can be ordered in a minimum scale of synthesis, which is suitable to stablish experimental procedures in an affordable approach. Other plausible types of probes to be used in this procedure are summarized in the Table: 1

Table 1. Main features of Affinity Plus and MGB probes.

| Type of probe | Hydrolysis Affinity Plus probe | Hydrolysis MGB |
|---|---|---|
| Oligo modification at 5' (Dye) | FAM™ | FAM™ |
| Oligo modification at 3' (Quencher) | Iowa Black® | MGB |
| Molecular description | Locked nucleic acids are modified RNA monomers. The "locked" part comes from a methylene bridge bond linking the 2' oxygen to the 4' carbon of the RNA pentose ring. The bridge bond fixes the pentose ring in the 3'-end conformation. | MGB moiety conjugation (*minor groove ligands*) at the 3'-end of the DNA strand. |
| Main advantages | High specificity.<br>High stability in short length probes.<br>Increase of melting temperature.<br>Flexible range adjustment of melting temperature through LNA sequences. | High specificity.<br>High stability in short length probes.<br>Increase of melting temperature. |

In other preferred embodiment of the present invention, the kit further comprises at least one forward primer comprising the nucleotide sequence SEQ ID NO: 49, preferably the forward primer consisting of the nucleotide sequence SEQ ID NO: 49.
SEQ ID NO: 49 5'-CGGGCGTTGNNNNNNNNNNNN-3'
In other more preferred embodiment, the forward primer has a length of 22 nucleotides comprising the nucleotide sequence SEQ ID NO: 2 or SEQ ID NO: 50, which sequences include a small tag [11 nucleotides, CTCGGGCGTTG (SEQ ID NO: 47), in the sequence SEQ ID NO: 2, or 9 nucleotides, CGGGCGTTG (SEQ ID NO: 51) in the sequence SEQ ID NO: 50] at the 5' end belonging to the species *Araucaria Scopolorum* (for avoiding any possible unspecific overlapping) and 11 nucleotides homologous with the targeted short length RNA in the sequence SEQ ID NO: 2 or 13 nucleotides homologous with the targeted short length RNA in the sequence SEQ ID NO: 50, the "NNNNNNNNNNN" region of the SEQ ID NO: 2 and the "NNNNNNNNNNNNN" region of the SEQ ID NO: 50.

SEQ ID NO: 2
5'-CTCGGGCGTTGNNNNNNNNNNN-3'

SEQ ID NO:50 5'-CGGGCGTTGNNNNNNNNNNNNN-3'

In other more preferred embodiment of the kit of the invention, the forward primer comprising the nucleotide sequence SEQ ID NO:2 or SEQ ID NO: 50, preferably the SEQ ID NO: 50.
In other more preferred embodiment of the kit of the invention, the forward primer consisting of the nucleotide sequence SEQ ID NO:2 or SEQ ID NO: 50, preferably the SEQ ID NO:50.
In other more preferred embodiment of the kit of the invention, the forward primer consisting of the nucleotide sequence SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46 and any combination thereof.
The term "universal reverse primer" or "universal probe" used herein refers to a reverse primer or probe which are total or partially complementary to the stem region of the stem loop primer and, this way, potentially any short length RNA, preferably miRNA, which would have been previously reverse-transcribed with this specific stem loop could be amplified.
The proposed reverse primer is universal, hybrid with de cDNA in the stem and loop regions which comprises the nucleotide sequence SEQ ID NO: 3.
SEQ ID NO: 3
3'-CAGCCTGGGACGTGGTC-5'
In other preferred embodiment of the present invention, the kit further comprises a universal reverse primer comprising the nucleotide sequence SEQ ID NO: 3.
In other preferred embodiment of the present invention, the universal reverse primer consisting of the nucleotide sequence SEQ ID NO: 3.

Reverse primers (SEQ ID NO: 4 and SEQ ID NO: 5) were both designed as 19 nucleotides length sequences, 9 nucleotides in SEQ ID NO: 4 or 7 nucleotides in SEQ ID NO: 5.

SEQ ID NO: 4
3'-CAGCCTGGGA<u>CGTGGTCTC</u>-5'

SEQ ID NO: 5
3'-TGCAGCCTGGGA<u>CGTGGTC</u>-5'

The underlined nucleotides in SEQ ID NO: 3, SEQ ID NO: 4 and SEQ ID NO: 5 fit with the stem region in the 5'tail of the stem loop and the additional 10 and 12 nucleotides of reverse primers have the same sequence composition of the loop.

In other more preferred embodiment of the kit of the invention, the universal reverse primer comprising the nucleotide sequence SEQ ID NO: 4 or SEQ ID NO:5.

In other more preferred embodiment of the kit of the invention, the universal reverse primer consisting of the nucleotide sequence SEQ ID NO: 4 or SEQ ID NO:5.

As mentioned before, the probe of the invention is universal for amplifying as many short length RNAs as required for the assay. The universal amplification probe overlaps exclusively with the cDNA region from the universal stem loop region (in bold in SEQ ID NO: 6 or in SEQ ID NO: 7).

In other preferred embodiment of the present invention, the kit further comprises a universal probe comprising the nucleotide sequence SEQ ID NO: 6, preferably consisting of the nucleotide sequence SEQ ID NO: 6.
SEQ ID NO: 6
**3'-CAACCGAGACCACG**CTTATGCA-5'

In other preferred embodiment of the present invention, the universal probe comprising the nucleotide sequence SEQ ID NO: 7, preferably consisting of the nucleotide sequence SEQ ID NO: 7.
SEQ ID NO: 7
3'- **CAACCGAGACCACG**CTTATGCAGCC-5'

The reverse transcription reaction can be performed with a first kit followed by quantitative PCR amplification with a second kit of the invention. Alternatively, both reverse transcription and quantitative PCR amplification can take place in the same kit of the invention.

The inventors have developed a short length RNA detection method based on the combination of the stem-loop of the invention and at least one specific forward primer with a universal reverse primer and a universal probe in a reverse-transcription quantitative PCR (RT-qPCR) system for the specific detection of nucleic acid sequences. This system only requires the design of a specific stem-loop and forward primer for each target short length RNA, while the universal reverse and probe can detect all short length RNAs (Figure 6).

So, other aspect of the invention relates to an *in vitro* method for detecting and/or amplifying at least one target short length RNA in an isolated biological sample of a subject comprising:

(i) reverse-transcribing the target short length RNA using a reverse transcriptase and the stem-loop, the composition or the kit of the invention, obtaining a double-stranded cDNA, and
(ii) performing a quantitative PCR (qPCR) using as a template the double-stranded cDNA obtained in (i) and

- at least one forward primer comprising the nucleotide sequence SEQ ID NO: 49, a universal reverse primer comprising the nucleotide sequence SEQ ID NO: 3 and/or a universal probe comprising the nucleotide sequence SEQ ID NO: 6, or
- the kit of the invention.

As used herein, the term "short length RNA" refers to are polymeric RNA molecules that are less than 200 nucleotides in length and are usually non-coding. Examples of short length RNA include, without limitation, microRNA (miRNA), Piwi-interacting RNA (piRNA), small interfering RNA (siRNA), small nuclear RNA (snRNA), small nucleolar RNA (snoRNA), small rDNA-derived RNA (srRNA), tRNA-derived small RNA (tsRNA) and Y RNA-derived small RNA (ysRNA).

In a preferred embodiment of the method of the invention, the target short length RNA is selected from the list consisting of: microRNA (miRNA), Piwi-interacting RNA (piRNA), small interfering RNA (siRNA), small nuclear RNA (snRNA), small nucleolar RNA (snoRNA), small rDNA-derived RNA (srRNA), tRNA-derived small RNA (tsRNA) and Y RNA-derived small RNA (ysRNA).

In a more preferred embodiment of the method of the invention, the target short length RNA is a miRNA.

As used herein, the term "miRNA" or "microRNA" refers to a small single-stranded non-coding RNA molecule (containing about 22 nucleotides) found in plants, animals and some viruses, that functions in RNA silencing and post-transcriptional regulation of gene expression. Therefore, they have been proposed as main regulators of messenger RNA expression, biological essential process related with several pathologies such cancer amount others. As used herein, the term "target miRNA" refers to a miRNA sequence that is sought to be amplified and/or quantified. Further, it will be appreciated that "target miRNA" can refer to the target miRNA itself, as well as surrogates thereof, for example amplification products, and native sequences.

Potentially the design of the method allows the detection of any miRNAs. The stem-loop for each miRNA contains at the 3'-end the first 6 specific nucleotides for each target miRNAs, maintaining the sequence of the stem-loop (SEQ ID NO: 1). The forward primer would also be specific for each target miRNA. The reverse and probe are universal for, potentially valid for detecting of all miRNAs.

In a preferred embodiment of the method of the invention, the target miRNA is selected from the list consisting of: miR 146a-5p (SEQ ID NO: 8), hsa-miR 155-5p (SEQ ID NO: 9), hsa-miR 21-5p (SEQ ID NO: 10), hsa-miR 194-2-5p (SEQ ID NO: 11), hsa-miR 223-3p (SEQ ID NO: 12), hsa-miR 192-5p (SEQ ID NO: 13), hsa-miR 23a-3p (SEQ ID NO: 14), hsa-miR 200c-3p (SEQ ID NO: 15), hsa-miR 22-3p (SEQ ID NO: 16), hsa-miR 16-5p (SEQ ID NO: 17), hsa-miR 191-5p (SEQ ID NO: 18), hsa-miR-200a-5p (SEQ ID NO: 19), hsa-miR-106a-5p (SEQ ID NO: 20) and any combination thereof.

In a preferred embodiment of the method of the invention, the stem-loop primer sequences of miRNA describe above is selected from the list consisting of: miR 146a-5p (SEQ ID NO: 21), hsa-miR 155-5p (SEQ ID NO: 22), hsa-miR 21-5p (SEQ ID NO: 23), hsa-miR 194-2-5p (SEQ ID NO: 24), hsa-miR 223-3p (SEQ ID NO: 25), hsa-miR 192-5p (SEQ ID NO: 26), hsa-miR 23a-3p (SEQ ID NO: 27), hsa-miR 200c-3p (SEQ ID NO: 28), hsa-miR 22-3p (SEQ ID NO: 29), hsa-miR 16-5p (SEQ ID NO: 30), hsa-miR 191-5p (SEQ ID NO: 31), hsa-miR-200a-5p (SEQ ID NO: 32), hsa-miR-106a-5p (SEQ ID NO: 33) and any combination thereof.

In a preferred embodiment of the method of the invention, the forward primer sequences of miRNA describe above is selected from the list consisting of: miR 146a-5p (SEQ ID NO: 34), hsa-miR 155-5p (SEQ ID NO: 35), hsa-miR 21-5p (SEQ ID NO: 36), hsa-miR 194-2-5p (SEQ ID NO: 37), hsa-miR 223-3p (SEQ ID NO: 38), hsa-miR 192-5p (SEQ ID NO: 39), hsa-miR 23a-3p (SEQ ID NO: 40), hsa-miR 200c-3p (SEQ ID NO: 41), hsa-miR 22-3p (SEQ ID NO: 42), hsa-miR 16-5p (SEQ ID NO: 43), hsa-miR 191-5p (SEQ ID NO: 44), hsa-miR-200a-5p (SEQ ID NO: 45), hsa-miR-106a-5p (SEQ ID NO: 46) and any combination thereof.

The specific sequences of these miRNAs are shown below, together with the designed specific stem-loop primers and specific forward primer sequences for each one of them (Table 2, 3 and 4). Sequences are available at the repository of miRNAs miRbase.

Table 2. Mature miRNAs sequence

| Target | SEQ ID NO | Sequence (5'-3') |
|---|---|---|
| hsa-miR-146a-5p | SEQ ID NO: 8 | UGAGAACUGAAUUCCAUGGGUU |
| hsa-miR-155-5p | SEQ ID NO: 9 | UUAAUGCUAAUCGUGAUAGGGGUU |
| hsa-miR-21-5p | SEQ ID NO: 10 | UAGCUUAUCAGACUGAUGUUGA |
| hsa-miR-194-2-5p | SEQ ID NO: 11 | UGUAACAGCAACUCCAUGUGGA |
| hsa-miR-223-3p | SEQ ID NO: 12 | UGUCAGUUUGUCAAAUACCCCA |
| hsa-miR-192-5p | SEQ ID NO: 13 | CUGACCUAUGAAUUGACAGCC |
| hsa-miR-23a-3p | SEQ ID NO: 14 | AUCACAUUGCCAGGGAUUUCC |
| hsa-miR-200c-3p | SEQ ID NO: 15 | UAAUACUGCCGGGUAAUGAUGGA |
| hsa-miR-22-3p | SEQ ID NO: 16 | AAGCUGCCAGUUGAAGAACUGU |
| hsa-miR-16-5p | SEQ ID NO: 17 | UAGCAGCACGUAAAUAUUGGCG |
| hsa-miR-191-5p | SEQ ID NO: 18 | CAACGGAAUCCCAAAAGCAGCUG |
| hsa-miR-200a-5p | SEQ ID NO: 19 | UAACACUGUCUGGUAACGAUGU |
| hsa-miR-106a-5p | SEQ ID NO: 20 | AAAAGUGCUUACAGUGCAGGUAG |

Table 3. Stem-loop primer sequences (target-specific region underlined)

| Target | SEQ ID NO | Stem-loop sequence (5'-3') |
|---|---|---|
| hsa-miR-146a-5p | SEQ ID NO: 21 | GTTGGCTCTGGTGCAGGGTCCGACG TATTCGCACCAGAGCCAAC<u>AACCCA</u> |

(continued)

| Target | SEQ ID NO | Stem-loop sequence (5'-3') |
|---|---|---|
| hsa-miR-155-5p | SEQ ID NO: 22 | GTTGGCTCTGGTGCAGGGTCCGACG TATTCGCACCAGAGCCAAC<u>AACCCC</u> |
| hsa-miR-21-5p | SEQ ID NO: 23 | GTTGGCTCTGGTGCAGGGTCCGACG TATTCGCACCAGAGCCAAC<u>TCAACA</u> |
| hsa-miR-194-2-5p | SEQ ID NO: 24 | GTTGGCTCTGGTGCAGGGTCCGACG TATTCGCACCAGAGCCAAC<u>TCCACA</u> |
| hsa-miR-223-3p | SEQ ID NO: 25 | GTTGGCTCTGGTGCAGGGTCCGACG TATTCGCACCAGAGCCAAC<u>TGGGGT</u> |
| hsa-miR-192-5p | SEQ ID NO: 26 | GTTGGCTCTGGTGCAGGGTCCGACG TATTCGCACCAGAGCCAAC<u>GGCTGT</u> |
| hsa-miR-23a-3p | SEQ ID NO: 27 | GTTGGCTCTGGTGCAGGGTCCGACG TATTCGCACCAGAGCCAAC<u>GGAAAT</u> |
| hsa-miR-200c-3p | SEQ ID NO: 28 | GTTGGCTCTGGTGCAGGGTCCGACG TATTCGCACCAGAGCCAAC<u>TCCATC</u> |
| hsa-miR-22-3p | SEQ ID NO: 29 | GTTGGCTCTGGTGCAGGGTCCGACG TATTCGCACCAGAGCCAAC<u>ACAGTT</u> |
| hsa-miR-16-5p | SEQ ID NO: 30 | GTTGGCTCTGGTGCAGGGTCCGACG TATTCGCACCAGAGCCAAC<u>CGCCAA</u> |
| hsa-miR-191-5p | SEQ ID NO: 31 | GTTGGCTCTGGTGCAGGGTCCGACG TATTCGCACCAGAGCCAAC<u>CAGCTG</u> |
| hsa-miR-200a-5p | SEQ ID NO: 32 | GTTGGCTCTGGTGCAGGGTCCGACG TATTCGCACCAGAGCCAAC<u>ACATCG</u> |
| hsa-miR-106a-5p | SEQ ID NO: 33 | GTTGGCTCTGGTGCAGGGTCCGACG TATTCGCACCAGAGCCAAC<u>CTACCT</u> |

Table 4. Forward primer sequences (target-specific region underlined)

| Target | SEQ ID NO | Sequence (5'-3') |
|---|---|---|
| hsa-miR-146a-5p | SEQ ID NO: 34 | CGGGCGTTG<u>TGAGAACTGAATT</u> |
| hsa-miR-155-5p | SEQ ID NO: 35 | CGGGCGTTG<u>TTAATGCTAATCG</u> |
| hsa-miR-21-5p | SEQ ID NO: 36 | CGGGCGTTG<u>TAGCTTATCAGAC</u> |
| hsa-miR-194-2-5p | SEQ ID NO: 37 | CGGGCGTTG<u>TGTAACAGCAACT</u> |
| hsa-miR-223-3p | SEQ ID NO: 38 | CGGGCGTTG<u>TGTCAGTTTGTCA</u> |
| hsa-miR-192-5p | SEQ ID NO: 39 | CGGGCGTTG<u>CTGACCTATGAAT</u> |
| hsa-miR-23a-3p | SEQ ID NO: 40 | CGGGCGTTG<u>ATCACATTGCCAG</u> |
| hsa-miR-200c-3p | SEQ ID NO: 41 | CGGGCGTTG<u>TAATACTGCCGGG</u> |

(continued)

| Target | SEQ ID NO | Sequence (5'-3') |
|---|---|---|
| hsa-miR-22-3p | SEQ ID NO: 42 | CGGGCGTTGAAGCTGCCAGTTG |
| hsa-miR-16-5p | SEQ ID NO: 43 | CGGGCGTTGTAGCAGCACGTAA |
| hsa-miR-191-5p | SEQ ID NO: 44 | CGGGCGTTGCAACGGAATCCCA |
| hsa-miR-200a-5p | SEQ ID NO: 45 | CGGGCGTTGTAACACTGTCTGG |
| hsa-miR-106a-5p | SEQ ID NO: 46 | CGGGCGTTGAAAAGTGCTTACA |

The target short length RNAs of the present invention can be derived or isolated from a biological sample of a subject, including without limitation, viruses, prokaryotes, eukaryotes, for example but not limited to plants, fungi, and animals.

In a preferred embodiment of the method of the invention, the subject is an animal, preferably a human.

The method could detect short length RNA in all types of samples where short length RNAs are expressed. Examples of isolated biological samples include, without limitation to, total blood, a tissue or liquid biopsy, lymph, bone marrow, amniotic fluid, hair, skin, semen, biowarfare agents, anal secretions, vaginal secretions, perspiration, saliva, buccal swabs, research samples generally, purified samples generally, cultured cells, and lysed cells. It will be appreciated that target short length RNA, preferably miRNA, can be isolated from samples using any of a variety of procedures known in the art, for example, the miRNeasy Mini Kit (Qiagen). However, miRNAs detection could be performed from RNA extracted with any standard protocol that would assure the recovery of small RNAs.

In a preferred embodiment of the method of the invention, the biological sample is selected from the list consisting of: feces, plasma, blood, cultures and lysed cells, tissues or biopsy from tissue, cervical mucus, vaginal secretions, saliva, buccal swabs, sperm and seminal plasm, amniotic fluid and any combination thereof.

As used herein, the term "amplifying" refers to any means by which at least a part of a target short length RNA, preferably miRNA, is reproduced, typically in a template-dependent manner, including without limitation, a broad range of techniques for amplifying nucleic acid sequences, either linearly or exponentially.

As used herein, the term "detection" refers to any of a variety of ways of determining the presence and/or quantity and/or identity of a target short length RNA, preferably miRNA.

The method of the invention for detecting and/or amplifying a target short length RNA is based on a Reverse Transcription Polymerase Chain Reaction (RT-PCR). The term "RT-PCR" or "Reverse Transcription Polymerase Chain Reaction" used herein refers to a modification of the standard PCR technique that can be used to amplify messenger RNA (mRNA). As a first step, isolated mRNA is converted to a complementary DNA (cDNA) molecule with an RNA-dependent DNA polymerase, also known as reverse transcriptase, during a process called reverse transcription.

As used herein, the term "reverse transcription" or "retrotranscription" refers to a process or reaction in molecular biology that involves the generation of a double-stranded deoxyribonucleic acid (DNA) strand called complementary DNA (cDNA) from a single-stranded ribonucleic acid (RNA), particularly an elongation reaction in which the 3' target-specific portion of a stem-loop primer is extended to form an extension reaction product comprising a strand complementary to the target short length RNA, preferably to the target miRNA.

Preferably, the reverse transcription reaction comprising a reverse transcriptase, where the 3' end of a stem-loop primer is extended. The term "reverse transcriptase" has been previously defined and said definition is equally valid for the present aspect as well as its embodiments.

The method of the invention comprises reverse-transcribing the target short length RNA using a reverse transcriptase and the stem-loop of the invention, the composition of the invention or the kit of the invention.

The "stem-loop", the "composition" and the "kit of the invention" have been previously defined and said definitions are equally valid for the present aspect as well as its embodiments.

The skilled person in the art can easily determine, for any given sample type, the reverse transcription reaction for reverse-transcribing the target short length RNA.

In other preferred embodiment of the method of the invention, the reverse transcription reaction of the step (i) is carried out between 5 and 100 minutes at a temperature ranges 15°C and 90°C (the range-ends are included).

In other more preferred embodiment of the method of the invention, the reverse transcription reaction of the step (i) is carried out by the followings step: 30 minutes at 16°C, 30 minutes at 42° C and 5 minutes at 85°C.

The reverse transcription reaction of the method of the invention is followed by an amplification qPCR reaction using the double-stranded cDNA obtained in step (i) as a template and:

- at least one forward primer comprising the nucleotide sequence SEQ ID NO: 49, a universal reverse primer comprising the nucleotide sequence SEQ ID NO: 3 and/or a universal probe comprising the nucleotide sequence SEQ ID NO: 6, or

- the kit of the invention.

The "forward primers", the "universal reverse primers", the "universal probe" and the "kit of the invention" have been previously defined and said definitions are equally valid for the present aspect as well as its embodiments.

In other preferred embodiment of the method of the invention, the forward primer comprising the nucleotide sequence SEQ ID NO: 2 or SEQ ID NO: 50, preferably the nucleotide sequence SEQ ID NO: 50.

In other more preferred embodiment of the method of the invention, the forward primer consisting of the nucleotide sequence SEQ ID NO: 2 or SEQ ID NO: 50, preferably the nucleotide sequence SEQ ID NO: 50.

In other preferred embodiment of the method of the invention, the universal reverse primer comprising the nucleotide sequence SEQ ID NO: 4 or SEQ ID NO: 5.

In other more preferred embodiment of the method of the invention, the universal reverse primer consisting of the nucleotide sequence SEQ ID NO: 4 or SEQ ID NO: 5.

As the skilled person in the art knows, different detector probes may distinguish between different target short length RNA. Examples of probes include, without limiting to, Affinity Plus probe, Taqman probe, and MGB probe, being the Affinity Plus the preferred one.

In other preferred embodiment of the method of the invention, the universal probe comprising the nucleotide sequence SEQ ID NO: 7.

In other more preferred embodiment of the method of the invention, the universal probe consisting of the nucleotide sequence SEQ ID NO: 7.

As the skilled person in the art knows, the amount of probe that gives a fluorescent signal in response to an excited light typically relates to the amount of nucleic acid produced in the amplification reaction, i.e., the amount of fluorescent signal is related to the amount of product created in the amplification reaction. Devices have been developed that can perform a thermal cycling reaction with compositions containing a fluorescent indicator, emit a light beam of a specified wavelength, read the intensity of the fluorescent dye, and display the intensity of fluorescence after each cycle; each of these functions can be performed by separate devices. Preferably, fluorescent signals can be detected and displayed during and/or after one or more thermal cycles, thus permitting monitoring of amplification products as the reactions occur in "real time."

As the skilled person in the art knows, it can be used the amount of amplification product and number of amplification cycles to calculate how much of the target nucleic acid sequence was in the sample prior to amplification and monitor the amount of amplification product after a predetermined number of cycles sufficient to indicate the presence of the target nucleic acid sequence in the sample. The skilled person in the art can easily determine, for any given sample type, the reaction conditions and how many cycles are sufficient to determine the presence of a given target short length RNA. As used herein, determining the presence of a short length RNA target can comprise identifying it, as well as optionally quantifying it.

In other preferred embodiment of the method of the invention, the amplification qPCR reaction of the step (ii) comprises:

1) one cycle at 95ºC for 10 minutes,

2) 40 cycles comprising 15 seconds at 95ºC and 1 minute at 60ºC per cycle, and

3) holding at 10ºC.

It is routine practice for the skilled person in the art to determinate the primer concentration for carrying out the method of the invention.

The optimal range concentration of forward and universal reverse primer is 0,1 µM to 2 µM, the universal probe concentration range is 0.1 µM to 2 µM and/or the stem-loop primer concentration range is 0.1 nM to 3 nM (all the end-range included).

In other preferred embodiment the forward and universal reverse primer concentration is 1,5 µM and 0,7 µM, respectively.

In other preferred embodiment the universal probe concentration is 0,8 µM and/or the stem-loop primer concentration is 1 nM.

Regarding the amplification conditions in the PCR amplification of the step (ii), in order to increase the sensitivity of the reaction, the method of the invention contemplates an intermediate hybridization step.

In other preferred embodiment of the method of the invention, the qPCR comprises an intermediate hybridization step, preferably wherein the temperature of the hybridization step is adjusted to the average melting temperature of the forward primer of each targeted short length RNA.

In other more preferred embodiment of the method of the invention, the amplification PCR reaction of the step (ii) comprises:

1) one cycle at 95ºC for 10 minutes,

2) 40 cycles, wherein each cycle comprises: 15 seconds at 95ºC, 1 minute at XºC and 1 minute at 60ºC, and

3) holding at 10ºC,

wherein the temperature XºC of the hybridization is adjusted to the average melting temperature of the specific forward primers of each targeted short length RNA.

It is routine practice for the skilled person in the art to calculate the average melting temperature (temperature X ºC) of the specific forward primers of each targeted short length RNA. The selected method for the average melting temperature calculations is the OligoAnalyzer™ Tool from IDT. In any case, these calculation tools respond to *"The Wallace Rule"*:

$$(X)\ Tm = 2(A+T) + 4(G+C)$$

As described above, the kit of the invention allows the detection of short length RNA. So other aspect of the invention refers to the use of the kit of the invention for *in vitro* short length RNA detection.

In a preferred embodiment of the use of the kit of the invention, the target short length RNA is selected from the list consisting of: microRNA (miRNA), Piwi-interacting RNA (piRNA), small interfering RNA (siRNA), small nuclear RNA (snRNA), small nucleolar RNA (snoRNA), small rDNA-derived RNA (srRNA), tRNA-derived small RNA (tsRNA) and Y RNA-derived small RNA (ysRNA).

In a more preferred embodiment of the use of the kit of the invention, the target short length RNA is a miRNA.

In a preferred embodiment of the use of the kit of the invention, the miRNA is selected from the list consisting of: miR 146a-5p (SEQ ID NO: 8), hsa-miR 155-5p (SEQ ID NO: 9), hsa-miR 21-5p (SEQ ID NO: 10), hsa-miR 194-2-5p (SEQ ID NO: 11), hsa-miR 223-3p (SEQ ID NO: 12), hsa-miR 192-5p (SEQ ID NO: 13), hsa-miR 23a-3p (SEQ ID NO: 14), hsa-miR 200c-3p (SEQ ID NO: 15), hsa-miR 22-3p (SEQ ID NO: 16), hsa-miR 16-5p (SEQ ID NO: 17), hsa-miR 191-5p (SEQ ID NO: 18), hsa-miR-200a-5p (SEQ ID NO: 19), hsa-miR-106a-5p (SEQ ID NO: 20) and any combination thereof.

## DESCRIPTION OF THE DRAWINGS

**Figure 1.** miRNAs amplification plots from stools samples using the method of the invention (hsa-miR 146a-5p, hsa-miR 155-5p, hsa-miR 21-5p samples, hsa-miR 194-2-5p, hsa-miR 223-3p, hsa-miR 192-5p, hsa-miR 200c-3p, hsa-miR 22-3p, hsa-miR 16-5p, hsa-miR 23-5p, hsa-miR 191-5p). Samples are analyzed in duplicates (sigmoidal curves) and negative controls (flat curves) for both the retrotranscription and qPCR steps are included. Fluorescence signal normalized to the baseline (ΔRn) is represented versus cycle of amplification.

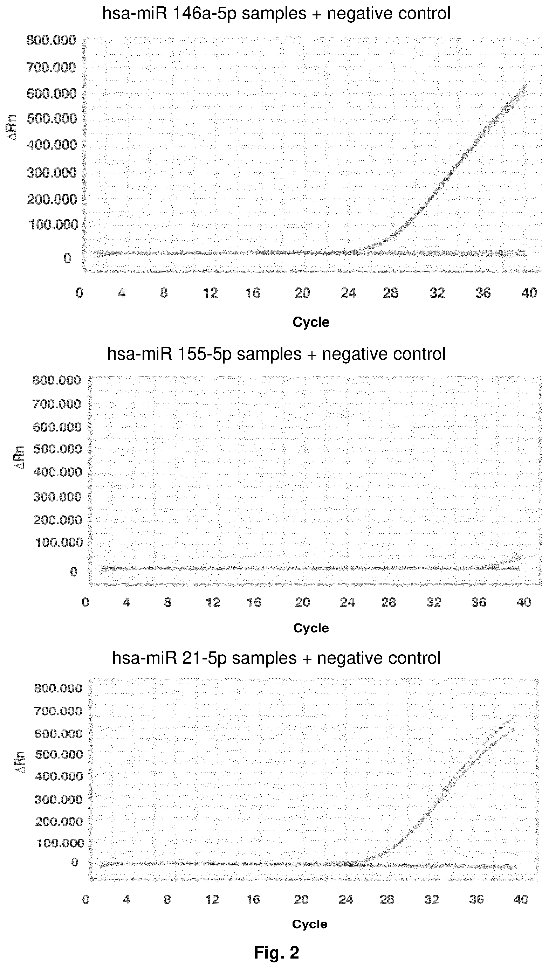

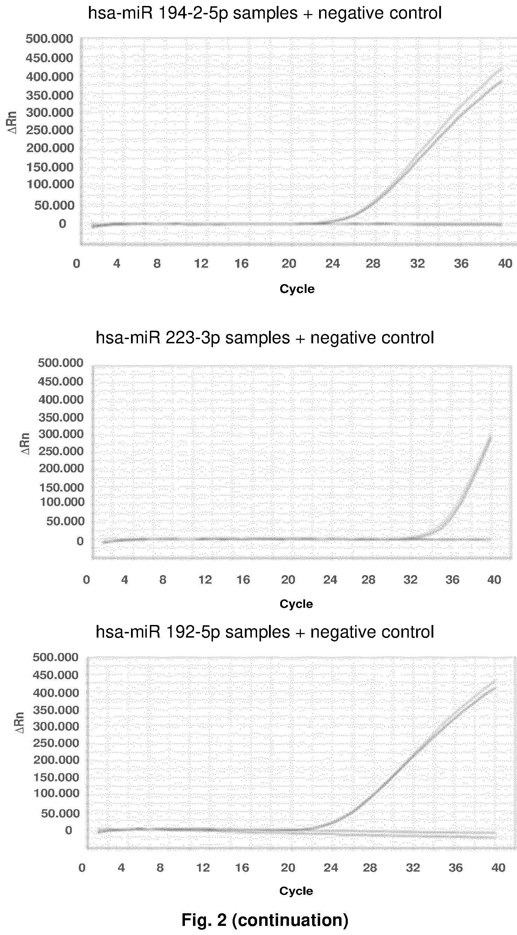

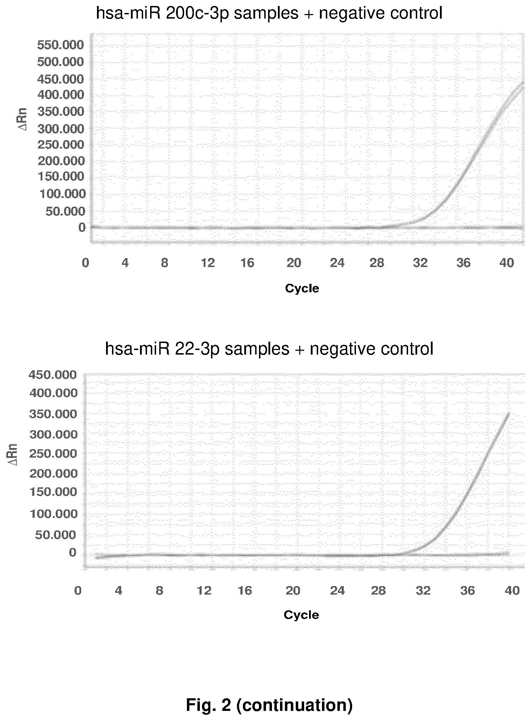

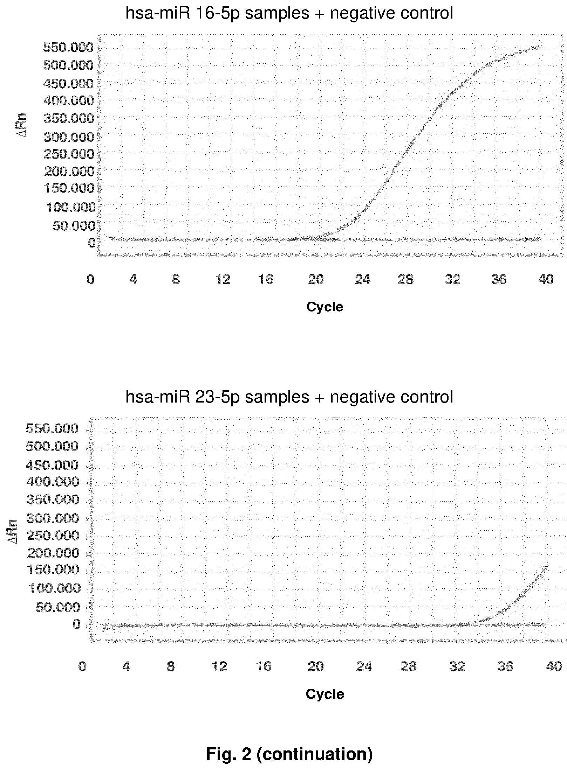

**Figure 2.** miRNAs amplification plots from Huh 7 cell line extracts using the proposed method (hsa-miR 146a-5p, hsa-miR 155-5p, hsa-miR 21-5p samples, hsa-miR 194-2-5p, hsa-miR 223-3p, hsa-miR 192-5p, hsa-miR 200c-3p, hsa-miR 22-3p, hsa-miR 16-5p, hsa-miR 23-5p, hsa-miR 191-5p). Samples are analyzed in duplicates (sigmoidal curves) and negative controls (flat curves) for both the retrotranscription and qPCR steps are included. Fluorescence signal normalized to the baseline (ΔRn) is represented versus cycle of amplification.

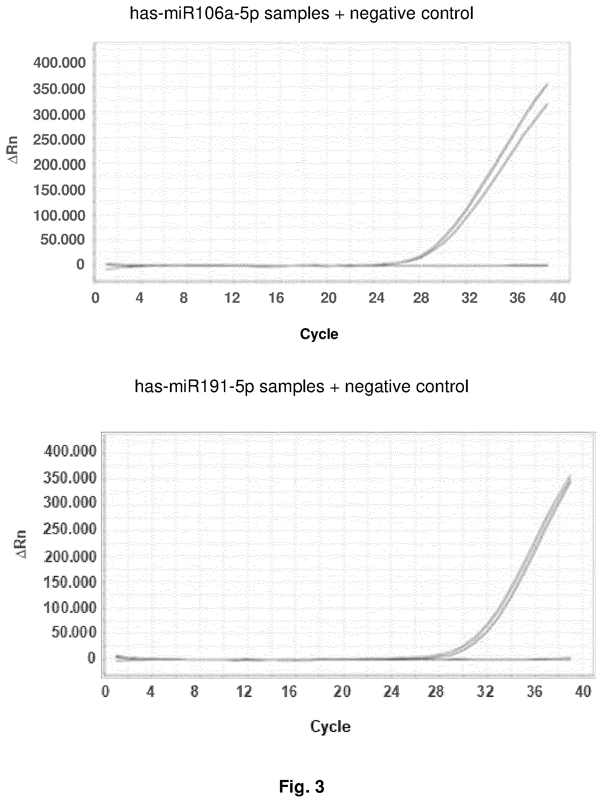

**Figure 3.** miRNAs amplification plots from serum samples using the proposed method. has-miR106a-5p and has-miR191-5p samples are analyzed in duplicates (sigmoidal curves) and negative controls (flat curves) for both the retrotranscription and qPCR steps are included. Fluorescence signal normalized to the baseline (ΔRn) is represented versus cycle of amplification.

**Figure 4.** Amplification plots comparison of hsa-miR-106a-5p from a control fecal sample using (A) the TaqMan Assay has-miR-106a-5pkit (Applied Biosystems) and (B) the proposed method. Fluorescence signal normalized to the baseline (ΔRn) is represented versus cycle of amplification.

**Figure 5.** RNA concentration and number of copies performance testing with the proposed method. (A) Lineal representation of the Has-miR-106a-5p amplification from 5, 10, 25 and 50 nanogrames of RNA and their corresponding cycle (Ct) values. B) Has-miR-106a-5p cDNA amplification curves from 6 to $6.10^7$ copies. No signal was detected in negative samples (NTC) Fluorescence signal normalized to the baseline (RFU) is represented versus cycle of amplification. (C) Lineal representation of the cDNA copy number versus Ct values. Linearity is observed from $6.10^3$ copies.

**Figure 6.** Schematic representation of the method. The system contains two specific primers (stem loop and forward primers) and two universal components (reverse primer and probe).

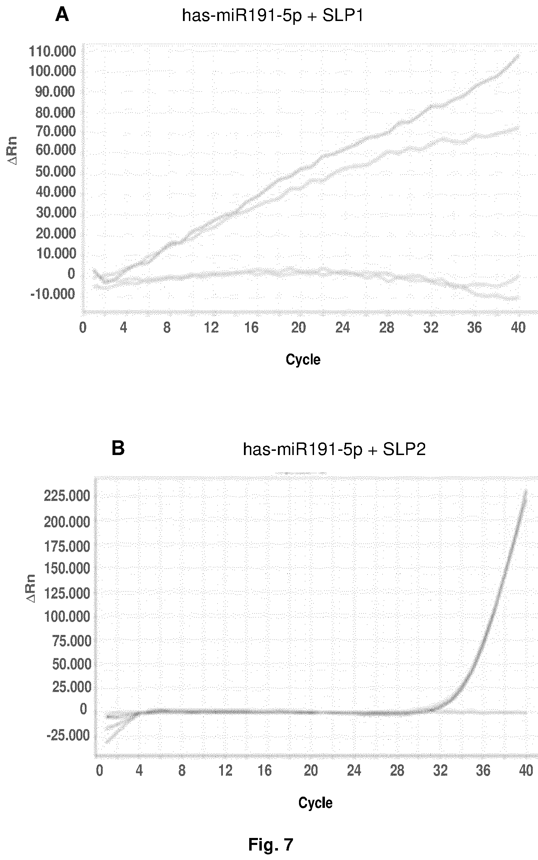

**Figure 7.** Has-miR-191-5p amplification plot comparison between an RT-qPCR experiment using (A) the non-modified stem-loop primer (SLP1) versus (B) the modified stem-loop primer (SLP2). Fluorescence signal normalized to the baseline (ΔRn) is represented versus cycle of amplification.

**Figure 8.** Amplification test in function of the quencher and probe length. A) Has-miR-106a-5p amplification using a short (22 nucleotides length) or a long (25 nucleotides length) probe with Affinity quencher. Also, a 25 nucleotides probe with MGB quencher was tested. B) Ct values representation of Has-miR-106a-5p amplification experiments with the 3 different probes. Fluorescence signal normalized to the baseline (ΔRn) is represented versus cycle of amplification

## EXAMPLES

**Retro-transcriptional (RT) stem loop primer design description**

The inventors have designed and optimized the stem loop primer (or hairpin primer) of the invention. As a first approach on the designing process, the stem loop primer was designed based on Garcia López, R., et al, 2018, "Caracterización funcional de microRNAs específicos de melón (C. melo L.) relacionados con la repuesta a estrés", Institute of Molecular and Cellular Biology of Plants. Polytechnic University of Valencia.

The sequence described in García López R., et al, 2018 is the following nucleotide sequence SEQ ID NO: 48, wherein the "NNNNNNN" is the miRNA-specific region with a 7 nucleotides length:
5'-GTTGGCTCTGGTGCAGGGTCCGAGGTATTCGCACCAGAGCCAACNNNNNNN-3'

After performing several initial experiments using this stem-loop primer, the inventors detected an unspecific overlap between the hairpin region and the specific region of some of the targeted miRNAs. In order to solve this problem, a modification was introduced in one of the bases located at the primer's hairpin (in 5'-3' sense, replacing the G at position 24 by a C, G24C underlined), obtaining the following nucleotide sequence SEQ ID NO: 1, wherein the "NNNNNNN" is the miRNA-specific region with a 6 nucleotides length:
5'-GTTGGCTCTGGTGCAGGGTCCGA<u>C</u>GTATTCGCACCAGAGCCAACNNNNNN - 3'

The stem loop is optimized for the performance of a pulsed retro-transcription reaction, followed by a standard PCR step and a final ligation process, i.e., a two-step RT-qPCR reaction, using a universal probe, a specific forward primer and a universal reverse primer.

After the modification of the stem-loop sequence (by replacing a G for a C and the 6 nucleotides miRNA-primer specific region), the non-specific amplification was solved, and the detection system showed an excellent performance amplifying all the miRNAs tested so far. In figure 7, the results showing the different performance of the non-modified (SLP1) (A) versus the modified stem-loop primer (SLP2) (B) used in RT-qPCR experiments to detect hsa-miR-191-5p in a feces sample. Curves shown in (A) are non-specific and present a random pattern, while (B) shows optimal amplification curves, high specificity (flat curves for the negative controls) and high reproducibility in all sample replicates.

**Optimization of the two-step amplification protocol**

qPCR oligos and probe design

Once the stem-loop primer sequence was established, the inventors proceeded to design the qPCR primers; based on Kramer M.F., 2011, Stem-loop RT-qPCR for miRNAs. Current Protocols in Molecular Biology Volume 95, Issue 1, p. 15.10.1-15.10.15.

The probe was ordered from IDT and it was designed as an Affinity Plus probe. This type of probe is based on the introduction of locked nucleotides (or LNAs) to improve the stability and sensitivity of the oligonucleotide. To this extent, they are similar to the MGB modifications, which also allow small probes reaching high specificity and stability levels. However, the Affinity Plus probe showed better amplification profiles than the probe with MGB quencher. Additionally, the efficiency of two Affinity Plus probes including 22 or 25 nucleotides, overlapping with the cDNA region from the universal stem loop region, was tested. The short probe with 22 nucleotides length showed the best amplification profile (Fig. 8) although both probes could efficiently amplify the target miRNA. As mentioned before, the probe of the invention is universal for amplifying as many miRNAs as required for the assay.

Oligo stocks were ordered lyophilized also from IDT (HPLC-purified) and were subsequently resuspended in nuclease-free water to reach a final concentration of 200 µM for the forward and universal primers and 100 µM of the probe and the stem-loop.

Stem-loop primer stock re-folding

One of the most critical parameters is the re-folding of the stem-loop into its stable structure. When this primer is subjected to high temperatures, some melting may occur. If it is incompletely folded, the reverse transcription of non-specific cDNA may occur. Where the miRNA of interest is rare, non-specific cDNA could exceed the specific cDNA and potentially contribute to non-specific PCR amplification. To perform this step, 100 µL aliquots are exposed to an initial step of 10 min at 95°C. Then, temperature is reduced slowly to 75°C and then kept hold at 75, 68, 65 and 62°C for an hour each. Finally, it is kept at 60°C for five to six hours (Table 5). This step is only required to be performed once, always assuring the storage temperature never peak above room temperature. (Kramer M.F., et al, 2011, Curr Protoc Mol Biol).

| Temperature | Time |
|---|---|
| 95ºC | 10 min |
| 75ºC (slow reduction) | 1 h |
| 68ºC | 1 h |
| 65ºC | 1 h |
| 62ºC | 1 h |
| 60ºC | 5-6 h |

Table 5. Thermocycler conditions

Retro-transcription step

miRNA is extracted from the selected sample. Afterward the RNA was retro-transcribed to synthesize a complementary DNA strand (cDNA) previously to perform quantitative PCR. The process of retro-transcription is performed with commercial kits which include retrotrascription enzymes (RT) such as Moloney Murine Leukemia Virus Reverse Transcriptase (Mu-MLV-RT) or Avian Myeloblastosis Virus Reverse Transcriptase (AMV-RT).

In the present invention, the MicroRNA Reverse Transcription Kit (Applied Biosystems) used contains reaction mixture: 10X RT buffer, dNTP mix w/dTTp (100M total), RNase inhibitor (20U/µL), and M-MLV defined as MultiScribe™ RT enzyme (50U/µL). Another RT kit was tested during the optimization steps: AccessQuick™ RT-PCR System (Promega), composed by a combination of *Tfl* DNA Polymerase, dNTPs, magnesium sulfate and reaction buffer. AMV RT enzyme is supplied separately. The defined experimental conditions are described in Table 6 for 50ng of RNA in a final volume of 7.5 µL. This reaction could be performed from 5 to 50ng of RNA.

Table 6. MicroRNA Reverse Transcription reagents. 5 µL reaction mixture (dNTPs, RT enzyme, 10x Buffer, RNAse inhibitor, stem-loop primer 5nM + Nuclease Free water) + 2.5 µL (50ng) of RNA = 7.5 µL of final volume.

| REAGENT | FINAL VOLUME |
|---|---|
| dNTPs 100 mM | 0.075 µL |
| RT enzyme | 0.5 µL |
| 10X Buffer | 0.75 µL |
| RNAse inhibitor | 0.095 µL |
| Stem loop primer 5nM | 1.5 µL |

(continued)

| REAGENT | FINAL VOLUME |
|---|---|
| Nuclease Free Water | 2.08 $\mu$L |
| Total Reaction Mixture | 5 $\mu$L |
| + | |
| RNA (50 ng) | 2.5 $\mu$L |
| Final Volume | 7.5 $\mu$L |

The 7,5 $\mu$L of the previously described reaction were incubated in a thermocycler for 30 minutes at 16ºC, 30 minutes at 42º C and finally 5 minutes at 85ºC (Table 7). The transcribed cDNA can be directly used or storage at -20ºC upon until use.

Table 7. RT temperature conditions and cycles.

| Cycles | Temperature | Time | Activity |
|---|---|---|---|
| 1 | 16°C | 30 minutes | Retrotranscription |
| 1 | 42°C | 30 minutes | |
| 1 | 85ºC | 5 minutes | Enzyme inactivation |

Quantitative PCR

After RNA retrotranscription of the RNA, the cDNA is amplified by quantitative PCR. Several taq polymerases systems can be used such as Go Taq probe Promega or NZYSpeedy qPCR Probe Master Mix DNA polymerase. The quantitative PCR reaction procedure is summarized in Table 8. The preferred kit selected for the qPCR step is the GoTaq Probe qPCR Master Mix (Promega), a ready-to-use stabilized 2X formulation containing GoTaq Hot Start Polymerase, MgCl2, dNTPs and proprietary reaction buffer. Another master mix used during the optimization steps is the NZYSpeedy qPCR Probe Master Mix (NZYtech) is provided as a 2× reaction mixture containing all components necessary for real-time PCR, including dNTPs, stabilizers and enhancers.

| REAGENT | FINAL VOLUME |
|---|---|
| Master mix (GoTaq Probe Promega) | 10 µL |
| 20 µM forward primer | 1.5 µL |
| 20 µM reverse primer | 0.7 µL |
| 10 µM universal probe | 1.6 µL |
| Nuclease Free Water | 3.7 µL |
| Total reaction mixture | 17.5 µL |
| + | |

| cDNA | 2,5 µL |
|---|---|

| Final volume | 20 µL |
|---|---|

Table 8. qPCR reaction mixture. 17.5 µL reaction mixture (Master mix, forward primer, reverse primer, universal probe and Nuclease Free Water) + 2.5 µL non-diluted cDNA = 20 µL of final volume.

**Conditions**

Quantitative PCR amplification cycles, temperature and time are summarized in table 9.

Table 9. qPCR temperature conditions. XᵒC temperature must be adjusted to the specific melting temperature of each specific forward primer.

| Cycles | Temperature | Time | Activity |
|---|---|---|---|
| 1 | 95°C | 10 minutes | Polymerase activation |
| 40 | 95°C | 15 seconds | Denaturalization |
| | XᵒC | 1 minute | Hybridization |
| | 60°C | 1 minute | Elongation Acquisition FAM-NFQMGB |
| 1 | 10°C | ∞ | Infinite holding |

The proposed reverse primer is universal, a 19 nucleotides length and it was designed based on the stem loop sequence (SEQ ID NO:4 or SEQ ID NO:5):

SEQ ID NO: 4
3'-CAGCCTGGGACGTGGTCTC-5

SEQ ID NO: 5
3'-TGCAGCCTGGGACGTGGTC-5'

The universal amplification probe, overlapping 22 or 25 nucleotides exclusively with the cDNA region from the universal stem loop region (SEQ ID NO:6 or SEQ ID NO:7):

SEQ ID NO: 6
3'-CAACCGAGACCACGCTTATGCA-5'

SEQ ID NO: 7
3'- CAACCGAGACCACGCTTATGCAGCC-5'

The Forward primer has a length of 22 nucleotides, including a small tag (11 or 9 nucleotides) at the 5' end belonging to the species *Araucaria Scopolorum* (avoiding any possible unspecific overlapping) and 11 or 13 nucleotides homologous with the targeted miRNA (SEQ ID NO: 2 or SEQ ID NO: 50):

SEQ ID NO: 2
5'-CTCGGGCGTTGNNNNNNNNNNN-3'

SEQ ID NO: 50
5'-CGGGCGTTGNNNNNNNNNNNNN-3'

Regarding the amplification conditions in qPCR, in order to increase the sensitivity of the reaction, the invention contemplates an intermediate hybridization step. The temperature (X$\underline{o}$C) of this step is adjusted to the average temperature of the specific forward primers of each targeted miRNA.

## Amplification system testing in human samples

So far, the proposed universal probe amplification system has been validated on a total of 40 human stool samples from patients with different types of tumors undergoing immunotherapy. It has also been tested on serum samples from patients with cancer and extracts from hepatocarcinoma cell cultures (Huh 7 cell line). 13 microRNAs have been tested (Table 10).

Table 10. microRNAs validated by the two-step universal probe amplification system

| miRNA | Huh-7 cells | Human stoos | Human serum |
|---|---|---|---|
| hsa-miR-146a-5p | ✓ | | |
| hsa-miR-155-5p | ✓ | ✓ | |
| hsa-miR-21-5p | ✓ | ✓ | |
| hsa-miR-194-2-5p | ✓ | ✓ | |
| hsa-miR-223-3p | ✓ | ✓ | |
| hsa-miR-192-5p | ✓ | ✓ | |
| hsa-miR-23a-3p | ✓ | ✓ | |
| hsa-miR-200c-3p | ✓ | ✓ | |
| hsa-miR-22-3p | ✓ | ✓ | |
| hsa-miR-16-5p | ✓ | ✓ | |
| hsa-miR-191-5p | ✓ | ✓ | ✓ |
| hsa-miR-200a-5p | ✓ | ✓ | |
| hsa-miR-106a-5p | ✓ | ✓ | ✓ |

The amplification replicates for both systems, validation in human stools samples (Fig 1) and in Huh-7 cells (Fig 2), are almost identical, showing a high level of reproducibility using real biological samples as a substrate. In all cases, the amplification of the negative controls was undetectable, reassuring the specificity of the technique.
Also, the method was tested in a small set of human serum samples, has-miR106a-5p and has-miR191-5p samples (Fig 3) were measured showing good amplification profiles. The variability amongst samples reflects the differential expression that the microRNAs show naturally in the tissue and cell lines tested (i.e., hsa-miR-155-5p and hsa-miR-23-5p in Fig. 1 and 2).

## Comparative amplification between the method of invention and a gold standard specific kit

As it was previously mentioned, several standard microRNA amplification kits include a specific probe for each miRNA detection obtaining high quality amplification profiles. Therefore, amplification profiles of the TaqMan™ MicroRNA Assay kit (Applied Biosystems) and the universal probe method of the invention were compared (Fig.4).
Hsa-miR106a-5p was tested on a selected stool sample proceeding by a healthy control (CS9). The universal detection kit allows to generate qPCR amplification profiles similar to the ones obtained from the Applied Biosystem kit, even though the commercial kit uses one specific probe per targeted miRNA. Once again, these results support a high specificity as one the main strengths of this novel miRNAs detection system (Fig 4 and Tables 11 to 14).

## Conditions

*Retrotranscription reaction*

Table 11. 10 $\mu$L reaction mix + 5 $\mu$L of RNA sample = 15 $\mu$ FV. Resulting cDNA is diluted 1:14 times nuclease free water (dilution A).

| REAGENT | FINAL VOLUME |
|---|---|
| Buffer RT | 1,5 $\mu$L |
| Enzima RT | 1 $\mu$L |
| Primer RT | 1 $\mu$L |
| dNTPs | 0,15 $\mu$L |
| RNAsa inhibitors | 0,19 $\mu$L |
| RNA | 10 ng |
| H2O | 6,16 $\mu$L |

Table 12. Retrotranscription reaction conditions.

| TEMPERATURE | TIME |
|---|---|
| 16ºC | 30 min |
| 42ºC | 30 min |
| 85ºC | 5 min |
| 4ºC | Infinite holding |

*Quantitative PCR*

Amplification using the gold standard system was performed according to the manufacturer instructions (detailed below):

Table 13. 14,7 reaction mix + 5,3 $\mu$L dilution A = 20 $\mu$L FV

| REAGENT | FINAL VOLUME |
|---|---|
| Gotaq mastermix (PROMEGA) | 10 $\mu$L |
| Taqman assay probe miR 106a (Applied Biosystems) | 1 $\mu$L |
| $H_2O$ | 3,7 $\mu$L |

Table 14. qPCR reaction conditions

| TEMPERATURE | TIME | CYCLES |
|---|---|---|
| 95ºC | 10 min | 1 |
| 95ºC | 15 seg | 40 |
| 60ºC | 1 min | |
| 4ºC | Infinite | Infinite holding |

Significantly the proposed method can be used to detect miRNAs from small amounts of RNA. The method amplifies 6 copies of miRNA cDNA reaching the linearity from $6 \times 10^3$. The minimal amount of RNA sample required in most of the commercial kit is at least 10ng, while the amount of 5ng of RNA is sufficient to detect miRNAs with the proposed method. Has-miR-106a-5p lineal amplification from 5, 10, 25 and 50 nanograms of RNA and their corresponding cycle (Ct) values are shown in figure 5. As it was already pointed out, due to the difficulty of obtaining some human tissues, this is an important advantage of the proposed method.

## EP 4 365 304 B1

**Claims**

1. A stem-loop primer comprising the nucleotide sequence SEQ ID NO: 1.

2. A composition comprising at least one stem-loop primer according to claim 1.

3. A kit for short length RNA detection comprising at least one stem-loop primer according to claim 1 or the composition according to claim 2.

4. Kit according to claim 3, further comprising a reverse transcriptase.

5. Kit according to claims 3 or 4, further comprising at least one forward primer comprising the nucleotide sequence SEQ ID NO: 49, preferably the nucleotide SEQ ID NO: 2 or SEQ ID NO: 50.

6. Kit according to any one of the claims 3 to 5, further comprising a universal reverse primer comprising the nucleotide sequence SEQ ID NO: 3 and/or a universal probe comprising the nucleotide sequence SEQ ID NO: 6, preferably the universal reverse primer comprising the nucleotide sequence SEQ ID NO: 4 or SEQ ID NO: 5 and/or the universal probe comprising the nucleotide sequence SEQ ID NO: 7.

7. An *in vitro* method for detecting and/or amplifying at least one target short length RNA in an isolated biological sample of a subject comprising:

    (i) reverse-transcribing the target short length RNA using a reverse transcriptase and the stem-loop primer according to claim 1, the composition according to claim 2 or the kit according to claim 3 or 4 obtaining a double-strand cDNA, and
    (ii) performing a quantitative PCR (qPCR) using as a template the double-strand cDNA obtained in (i) and

    - at least one forward primer comprising the nucleotide sequence SEQ ID NO: 49, preferably the nucleotide sequence SEQ ID NO: 2 or SEQ ID NO: 50; a universal reverse primer comprising the nucleotide sequence SEQ ID NO: 3, preferably the nucleotide sequence SEQ ID NO: 4 or SEQ ID NO: 5; and/or a universal probe comprising the nucleotide sequence SEQ ID NO: 6, preferably the nucleotide sequence SEQ ID NO: 7, or
    - the kit according to claim 5 or 6.

8. The method according to claim 7, wherein the qPCR comprises an intermediate hybridization step, preferably wherein the temperature of the hybridization step is adjusted to the average temperature of the forward primer of each targeted short length RNA.

9. The method according to claim 7 or 8, wherein the target short length RNA is a miRNA.

10. The method according to claim 9, wherein the miRNA is selected from the list consisting of: SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20 and any combination thereof.

11. The method according to any one of the claims 7 to 10, wherein the stem-loop primer sequence is selected from the list consisting of: SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33 and any combination thereof.

12. The method according to any one of the claims 7 to 11, wherein the forward primer sequence is selected from the list consisting of: SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46 and any combination thereof.

13. The method according to any one of the claims 7 to 12, wherein the forward and universal reverse primer concentration is 1,5 $\mu$M and 0,7 $\mu$M respectively, the universal probe concentration is 0,8 $\mu$M and/or the stem-loop primer concentration is 1nM.

14. Use of the kit according to any of the claims 3 to 6 for *in vitro* short length RNA detection.

**15.** Use according to claim 14, wherein the short length RNA is a miRNA.


**Patentansprüche**

**1.** Stem-Loop-Primer, der die Nukleotidsequenz SEQ ID NO: 1 umfasst.

**2.** Zusammensetzung, die mindestens einen Stem-Loop-Primer nach Anspruch 1 umfasst.

**3.** Kit für den Nachweis kurzer RNA, das mindestens einen Stem-Loop-Primer nach Anspruch 1 oder die Zusammensetzung nach Anspruch 2 umfasst.

**4.** Kit nach Anspruch 3, das ferner eine reverse Transkriptase umfasst.

**5.** Kit nach Anspruch 3 oder 4, das ferner mindestens einen Vorwärtsprimer mit der Nukleotidsequenz SEQ ID NO: 49, vorzugsweise dem Nukleotid SEQ ID NO: 2 oder SEQ ID NO: 50, umfasst.

**6.** Kit nach einem der Ansprüche 3 bis 5, das ferner einen universellen Rückwärtsprimer mit der Nukleotidsequenz SEQ ID NO: 3 und/oder eine Universalsonde mit der Nukleotidsequenz SEQ ID NO: 6 umfasst, wobei der universelle Rückwärtsprimer vorzugsweise die Nukleotidsequenz SEQ ID NO: 4 oder SEQ ID NO: 5 und/oder die Universalsonde die Nukleotidsequenz SEQ ID NO: 7 umfasst.

**7.** *In-vitro-Verfahren* zum Nachweis und/oder zur Amplifikation mindestens einer kurzen Ziel-RNA in einer isolierten biologischen Probe eines Individuums, umfassend:

(i) Reverse Transkription der kurzen Ziel-RNA unter Verwendung einer reversen Transkriptase und des Stem-Loop-Primers nach Anspruch 1, der Zusammensetzung nach Anspruch 2 oder des Kits nach Anspruch 3 oder 4 unter Erhalt einer doppelsträngigen cDNA, und
(ii) Durchführung einer quantitativen PCR (qPCR) unter Verwendung der in (i) erhaltenen doppelsträngigen cDNA als Matrize und

- mindestens einen Vorwärtsprimer, der die Nukleotidsequenz SEQ ID NO: 49, vorzugsweise die Nukleotidsequenz SEQ ID NO: 2 oder SEQ ID NO: 50 umfasst; einen universellen Rückwärtsprimer, der die Nukleotidsequenz SEQ ID NO: 3, vorzugsweise die Nukleotidsequenz SEQ ID NO: 4 oder SEQ ID NO: 5 umfasst; und/oder eine universelle Sonde, die die Nukleotidsequenz SEQ ID NO: 6, vorzugsweise die Nukleotidsequenz SEQ ID NO: 7 umfasst, oder
- das Kit nach Anspruch 5 oder 6.

**8.** Verfahren nach Anspruch 7, wobei die qPCR einen Hybridisierungszwischenschritt umfasst, wobei die Temperatur des Hybridisierungsschritts vorzugsweise an die Durchschnittstemperatur des Vorwärtsprimers jeder kurzen Ziel-RNA angepasst wird.

**9.** Verfahren nach Anspruch 7 oder 8, wobei die kurze Ziel-RNA eine miRNA ist.

**10.** Verfahren nach Anspruch 9, wobei die miRNA ausgewählt ist aus der Liste bestehend aus: SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20 und jeder Kombination davon.

**11.** Verfahren nach einem der Ansprüche 7 bis 10, wobei die Stem-Loop-Primersequenz ausgewählt ist aus der Liste bestehend aus: SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33 und jeder Kombination davon.

**12.** Verfahren nach einem der Ansprüche 7 bis 11, wobei die Vorwärtsprimersequenz ausgewählt ist aus der Liste bestehend aus: SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46 und jeder Kombination davon.

**13.** Verfahren nach einem der Ansprüche 7 bis 12, wobei die Konzentration des Vorwärts- bzw. des universellen Rückwärtsprimers 1,5 μM bzw. 0,7 μM, die Konzentration der universellen Sonde 0,8 μM und/oder die Konzentration des Stem-Loop-Primers 1 nM beträgt.

**14.** Verwendung des Kits nach einem der Ansprüche 3 bis 6 zum *In-vitro*-Nachweis der kurzen RNA.

**15.** Verwendung nach Anspruch 14, wobei die kurze RNA eine miRNA ist.

**Revendications**

**1.** Amorce tige-boucle comprenant la séquence nucléotidique SEQ ID N° : 1.

**2.** Composition comprenant au moins une amorce tige-boucle selon la revendication 1.

**3.** Kit pour la détection d'ARN de courte longueur comprenant au moins une amorce tige-boucle selon la revendication 1 ou la composition selon la revendication 2.

**4.** Kit selon la revendication 3, comprenant en outre une transcriptase inverse.

**5.** Kit selon les revendications 3 ou 4, comprenant en outre au moins une amorce sens comprenant la séquence nucléotidique SEQ ID N° : 49, de préférence le nucléotide SEQ ID N° : 2 ou SEQ ID N° : 50.

**6.** Kit selon l'une quelconque des revendications 3 à 5, comprenant en outre une amorce antisens universelle comprenant la séquence nucléotidique SEQ ID N° : 3 et/ou une sonde universelle comprenant la séquence nucléotidique SEQ ID N° : 6, de préférence, l'amorce antisens universelle comprenant la séquence nucléotidique SEQ ID N° : 4 ou SEQ ID N° : 5 et/ou la sonde universelle comprenant la séquence nucléotidique SEQ ID N° : 7.

**7.** Procédé *in vitro* pour détecter et/ou amplifier au moins un ARN de courte longueur cible dans un échantillon biologique isolé d'un sujet comprenant :

(i) la transcription inverse de l'ARN de courte longueur cible à l'aide d'une transcriptase inverse et de l'amorce tige-boucle selon la revendication 1, de la composition selon la revendication 2 ou du kit selon la revendication 3 ou 4 ce qui permet d'obtenir un ADNc double brin et
(ii) la mise en œuvre d'une PCR quantitative (qPCR) à l'aide en tant que matrice de l'ADNc double brin obtenu en (i) et

- d'au moins une amorce sens comprenant la séquence nucléotidique SEQ ID N° : 49, de préférence la séquence nucléotidique SEQ ID N° : 2 ou SEQ ID N° : 50 ; d'une amorce antisens universelle comprenant la séquence nucléotidique SEQ ID N° : 3, de préférence la séquence nucléotidique SEQ ID N° : 4 ou SEQ ID N° : 5 ; et/ou d'une sonde universelle comprenant la séquence nucléotidique SEQ ID N° : 6, de préférence la séquence nucléotidique SEQ ID N° : 7 ou
- du kit selon la revendication 5 ou 6.

**8.** Procédé selon la revendication 7, dans lequel la qPCR comprend une étape d'hybridation intermédiaire, de préférence dans lequel la température de l'étape d'hybridation est ajustée à la température moyenne de l'amorce sens de chaque ARN de courte longueur ciblé.

**9.** Procédé selon la revendication 7 ou 8, dans lequel l'ARN de courte longueur cible est un miARN.

**10.** Procédé selon la revendication 9, dans lequel le miARN est choisi dans la liste constituée de : SEQ ID N° : 8, SEQ ID N° : 9, SEQ ID N° : 10, SEQ ID N° : 11, SEQ ID N° : 12, SEQ ID N° : 13, SEQ ID N° : 14, SEQ ID N° : 15, SEQ ID N° : 16, SEQ ID N° : 17, SEQ ID N° : 18, SEQ ID N° : 19, SEQ ID N° : 20 et une quelconque combinaison de celles-ci.

**11.** Procédé selon l'une quelconque des revendications 7 à 10, dans lequel la séquence d'amorce tige-boucle est choisie dans la liste constituée de : SEQ ID N° : 21, SEQ ID N° : 22, SEQ ID N° : 23, SEQ ID N° : 24, SEQ ID N° : 25, SEQ ID N° : 26, SEQ ID N° : 27, SEQ ID N° : 28, SEQ ID N° : 29, SEQ ID N° : 30, SEQ ID N° : 31, SEQ ID N° : 32, SEQ ID N° : 33 et une quelconque combinaison de celles-ci.

**12.** Procédé selon l'une quelconque des revendications 7 à 11, dans lequel la séquence d'amorce sens est choisie dans la liste constituée de : SEQ ID N° : 34, SEQ ID N° : 35, SEQ ID N° : 36, SEQ ID N° : 37, SEQ ID N° : 38, SEQ ID N° : 39, SEQ ID N° : 40, SEQ ID N° : 41, SEQ ID N° : 42, SEQ ID N° : 43, SEQ ID N° : 44, SEQ ID N° : 45, SEQ ID N° : 46 et une quelconque combinaison de celles-ci.

**13.** Procédé selon l'une quelconque des revendications 7 à 12, dans lequel la concentration de l'amorce sens et de l'amorce antisens universelle est de 1,5 µM et 0,7 µM respectivement, la concentration de la sonde universelle est de 0,8 µM et/ou la concentration de l'amorce tige-boucle est de 1 nM.

**14.** Utilisation du kit selon l'une quelconque des revendications 3 à 6 pour la détection *in vitro* d'ARN de courte longueur.

**15.** Utilisation selon la revendication 14, dans laquelle l'ARN de courte longueur est un miARN.

hsa-miR 146a-5p samples + negative control

hsa-miR 155-5p samples + negative control

hsa-miR 21-5p samples + negative control

**Fig. 1**

Fig. 1 (continuation)

hsa-miR 200c-3p samples + negative control

hsa-miR 22-3p samples + negative control

hsa-miR 16-5p samples + negative control

Fig. 1 (continuation)

hsa-miR 23-5p samples + negative control

hsa-miR 191-5p samples + negative control

**Fig. 1 (continuation)**

Fig. 2

hsa-miR 194-2-5p samples + negative control

hsa-miR 223-3p samples + negative control

hsa-miR 192-5p samples + negative control

Fig. 2 (continuation)

hsa-miR 200c-3p samples + negative control

hsa-miR 22-3p samples + negative control

**Fig. 2 (continuation)**

## hsa-miR 16-5p samples + negative control

## hsa-miR 23-5p samples + negative control

**Fig. 2 (continuation)**

EP 4 365 304 B1

has-miR106a-5p samples + negative control

has-miR191-5p samples + negative control

**Fig. 3**

**A)** hsa-miR 106a-5p samples + negative control

**B)** hsa-miR 106a-5p samples + negative control

**Fig. 4**

**A**

**B**

**Fig. 5**

**C**

$y = -1,6104x + 31,277$
$R^2 = 0,9899$

Fig. 5 (continuation)

**miRNA**

**Stem loop primer**

Reverse transcription/ Quantitative PCR

3′                                              5′

**Forward primer**

5′                                              3′

**Reverse primer**

**Q**     **Universal probe**     **F**

Fig. 6

**A**      has-miR191-5p + SLP1

**B**      has-miR191-5p + SLP2

**Fig. 7**

**A**     has-miR106a-5p samples + negative control

**B**

**Fig. 8**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 113999896 A

**Non-patent literature cited in the description**

- **CHEN C. et al.** *Nucleic Acids Research*, 2005, vol. 33 (20)
- **KRAMER M.F. et al.** *Curr Protoc Mol Biol*, 2011
- **ULRIKE JUNG et al.** *RNA journal*, 2013, vol. 19 (12)
- **VARKONYI-GASIC et al.** *Plant Methods*, 2007, vol. 3 (12)
- Caracterización funcional de microRNAs específicos de melón (C. melo L.) relacionados con la repuesta a estrés. **GARCIA LÓPEZ, R. et al.** Institute of Molecular and Cellular Biology of Plants. Polytechnic University of Valencia, 2018
- **KRAMER M.F.** Stem-loop RT-qPCR for miRNAs. *Current Protocols in Molecular Biology*, 2011, vol. 95 (1), 15101-151015